(19) Europäisches Patentamt
European Patent Office
Office européen des brevets

(11) **EP 1 806 140 A1**

(12) **EUROPEAN PATENT APPLICATION**
published in accordance with Art. 158(3) EPC

(43) Date of publication:
**11.07.2007 Bulletin 2007/28**

(51) Int Cl.:
**A61K 33/44** (2006.01)        **A61P 3/10** (2006.01)
**A61P 25/00** (2006.01)

(21) Application number: **05785711.2**

(22) Date of filing: **21.09.2005**

(86) International application number:
**PCT/JP2005/017356**

(87) International publication number:
**WO 2006/033341 (30.03.2006 Gazette 2006/13)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **22.09.2004 JP 2004275733**

(71) Applicant: **Kureha Corporation
Tokyo 103-8552 (JP)**

(72) Inventors:
• **HIGUCHI, Yuusuke,
Biomedical Research Labs.
Tokyo 169-8503 (JP)**
• **OBARA, Kazumi,
Biomedical Research Labs.
Tokyo 169-8503 (JP)**
• **NISHIJIMA, Fuyuhiko,
Biomedical Research Labs.
Tokyo 169-8503 (JP)**

(74) Representative: **HOFFMANN EITLE
Patent- und Rechtsanwälte
Arabellastrasse 4
81925 München (DE)**

(54) **THERAPEUTIC OR PREVENTIVE AGENT FOR DIABETIC NEUROPATHY**

(57)     An agent effective for treating or preventing a diabetic neuropathy, which is a diabetic complication, is provided. The agent comprises a spherical activated carbon as an effective ingredient.

**EP 1 806 140 A1**

**Description**

TECHNICAL FIELD

The present invention relates to an agent for treating or preventing a diabetic neuropathy. The agent for treating or preventing a diabetic neuropathy according to the present invention is effective in the treating or preventing of a diabetic neuropathy, which is one of diabetic complications.

BACKGROUND ART

Diabetes is one of the diseases which have been remarkably increased in the number of the patients suffering therefrom in Japan recently. According to a research on an actual survey of diabetes by the Health and Welfare Ministry in 1997, the estimated number of the patients was 6,900 thousand. The number of such patients is almost the same as that of patients suffering from hyperlipemia or hypertension, and will certainly increase, and it is estimated that the current number of such patients could be doubled or tripled by 2010. Most important in the treatments of diabetes is a favorable control of a blood glucose level, and a means of suppressing the onset or the development of complications such as retinopathy, neurosis, nephropathy, peripheral circulation defect, or skin ulcers.

Insulin or a sulfonium urea compound has hitherto been used as a therapeutic medicament for diabetes, and it is known that they can effectively cause a drop a blood glucose level. Nonetheless, a complete cure of diabetes is difficult even by the above therapeutic medicament for diabetes. The prolongation of the suffering term has an onset of various complications, such as diabetic neurosis, as well as diabetic retinopathy, diabetic nephropathy, or diabetic peripheral circulation defect.

In diabetic neurosis, segmeutal demyelination of Schwann cells is remarkably observed. In the patients having a diabetic neurosis, hyperplasia or an obstruction of a basement membrane of a neurotrophic vascular is observed. It is believed that such an angiopathy causes a lack of a blood flow, and a subsequent neuropathy.

A therapeutic medicament for diabetic neuropathy is developed separately from a therapeutic medicament for diabetes. For example, an agent for treating or preventing a diabetic complication, comprising a thianaphthene derivative as an effective ingredient (Patent Reference No. 1), an agent for treating or preventing a diabetic complication, comprising an activator of a potassium channel as an effective ingredient (Patent Reference No. 2), an agent for treating or preventing a diabetic complication, comprising a postprandial hypoglycemic agent as an effective ingredient (Patent Reference No. 3), and the like are proposed.

However, it appears that there is no method gaining an international consensus as a method for treating such a diabetic neuropathy except for a strict control of a blood glucose level. Further, it is difficult for a patient suffering from diabetes to maintain a blood glucose level kinetics as in a healthy person, and it is impossible to completely inhibit an onset or a development of neuropathy only by the strictest possible control of a blood glucose level (for example, Non-Patent Reference No. 1).

Patent Reference No. 4 discloses an anti-diabetes agent comprising a spherical activated carbon as an effective ingredient. It also discloses that a blood glucose level was lowered 4 weeks later and 6 weeks later after orally administering a spherical activated carbon to rats with an elevated blood glucose level induced by an administration of streptozotocin, type 1 diabetes model rats, whereas a hypoglycemia was not caused in normal rats to which a spherical activated carbon was orally administered.

However, it is not known whether the spherical activated carbon is effective against a diabetic complication, in particularly a diabetic neuropathy.

[Patent Reference No. 1] Japanese Unexamined Patent Publication (Kokai) No. 7-223955
[Patent Reference No. 2] Japanese Unexamined Patent Publication (Kokai) No. 11-106352
[Patent Reference No. 3] WO01/068136
[Patent Reference No. 4] Japanese Unexamined Patent Publication (Kokai) No. 6-298653
[Non-Patent Reference No. 1] Jiro NAKAMURA, Treatment of diabetic neuropathy, Supplementary volume of "Igaku-no-Ayumi (Journal of Clinical and Experimental Medicine), Tonyobyo-Taishashokogun (Diabetes and Metabolic syndrome), State of Arts, 2004-2006", November 29, 2003, pp. 467-470, Ishiyaku Publishers, Inc.

DISCLOSURE OF THE INVENTION

PROBLEMS TO BE SOLVED BY THE INVENTION

During research into the development of a therapeutic medicament for diabetic neuropathy, the inventors of the present invention orally fed a spherical activated carbon to GK rats (Goto-Kakizaki rats), which are known as a spontaneous type 2 diabetic model rat and surprisingly found that a motor nerve conduction velocity (MNCV) was improved although a blood glucose level was not lowered, and thus the spherical activated carbon was effective in the treatment of a diabetic neuropathy.

At the present time, the reasons why, although the spherical activated carbon exhibits the effect to drop a blood glucose level in type 1 diabetes model rats (the Patent Reference No. 4) it shows a completely different behavior in spontaneous type 2 diabetic model rats, are not sufficiently elucidated. The GK rat, which is a spontaneous type 2 diabetic model rat, is in a more progressive pathological stage of diabetes in comparison with a type 1 diabetes model rat by an administration of streptozotocin. Therefore, one of the reasons for the above is believed to be that the GK rat is exposed at a high blood glucose level for a longer term.

Further, the above new finding that an oral administration of the spherical activated carbon is effective in a diabetic neuropathy, in spite of an inability to cause a drop in blood glucose level by an oral administration of the spherical activated carbon to a GK rat, i.e., a spontaneous type 2 diabetic model rat, suggests the presence of an unknown neuropathic factor other than a high blood glucose level. It is also surprising that the spherical activated carbon is effective in the removal of such a neuropathic factor other than a high blood glucose level.

The present invention is based on the above findings.

MEANS FOR SOLVING THE PROBLEMS

Accordingly, the present invention relates to an agent for treating or preventing a diabetic neuropathy, comprising a spherical activated carbon as an effective component.

According to a preferred embodiment of the agent of the present invention for treating or preventing a diabetic neuropathy, the agent is for an oral administration.

According to another preferred embodiment of the agent of the present invention for treating or preventing a diabetic neuropathy, a particle size of the spherical activated carbon is 0.01 to 2 mm.

According to still another preferred embodiment of the agent of the present invention for treating or preventing a diabetic neuropathy, the agent is for ameliorating a delay of a motor nerve conduction velocity.

EFFECTS OF THE INVENTION

The agent for treating or preventing a diabetic neuropathy according to the present invention is effective for treating or preventing a diabetic neuropathy, which is a diabetic complication. A repetitive oral administration does not cause toxicity, or harmful side effects such as constipation.

In general, a therapeutic drug for diabetes is not effective for diabetic complications, and thus, a therapeutic drug for diabetes and a therapeutic drug for diabetic complications are developed separately to each other. However, as mentioned above, a spherical activated carbon, which is an effective ingredient of the agent for treating or preventing a diabetic neuropathy according to the present invention, is effective for dropping a blood glucose level in an initial stage of diabetes, and although it cannot drop a blood glucose level in a prolonged stage of diabetes, it is also effective for a diabetic neuropathy. Therefore, a spherical activated carbon may be an effective agent for treating or preventing diabetes and complications thereof.

BEST MODE FOR CARRYING OUT THE INVENTION

The spherical activated carbon which may be used as the active ingredient of the medicament according to the present invention is not particularly limited, so long as it is a medical spherical activated carbon. As the spherical activated carbon, a spherical activated carbon for an oral administration, i.e., a spherical activated carbon which can be used for medical and internal use is preferable. The particle size of the spherical activated carbon is preferably 0.01 to 2 mm, more preferably 0.05 to 2 mm, most preferably 0.05 to 1 mm.

As the spherical activated carbon, for example, spherical activated carbons disclosed in Japanese Unexamined Patent Publication (Kokai) No. 11-292770 (Japanese Patent No. 3,522,708) or Japanese Unexamined Patent Publication (Kokai) No. 2002-308785 (Japanese Patent No. 3,522,708) may be used. Hereinafter, the spherical activated carbon disclosed in JP11-292770 will be first explained, and then the spherical activated carbon disclosed in JP2002-308785 will be explained.

The spherical activated carbon disclosed in JP11-292770 is a spherical activated carbon having a diameter of preferably 0.05 to 2 mm, more preferably 0.1 to 1 mm. A specific surface area thereof is preferably 500 to 2000 $m^2$/g, more preferably 700 to 1500 $m^2$/g. Further, a volume of pores having a pore radius of 100 to 75000 angstrom is preferably 0.01 to 1 mL/g, more preferably 0.05 to 0.8 mL/g. In this connection, the specific surface area is measured by a methanol adsorption method using an automatic adsorption measuring apparatus. The pore volume is measured by a mercury press-injection porosimeter. The spherical activated carbon has advantages in that it can be used as a dose without scattering, and that constipation is not caused by a repetitive administration, in comparison with a powdery activated carbon.

The shape of the spherical activated carbon is an important factor, and a substantially spherical shape is most important. Among known spherical activated carbons, a spherical activated carbon prepared from a petroleum pitch, as described below, is most preferable, because it is close to a completely spherical shape.

In the manufacture of the spherical activated carbon disclosed in JP11-292770, any starting materials for an active carbon, such as sawdust, coal, coconut shell, petroleum or coal pitches, or organic synthetic polymers, may be used. The spherical activated carbon may be produced, for example, by carbonizing the starting material and activating the carbonized substance. The activation may be performed by, for example, a steam-activation method, a chemical activation method, an air-activation method, or a $CO_2$ activation method, so long as a medically acceptable purity is maintained.

As the spherical activated carbon disclosed in JP11-292770, there may be mentioned, for example, a granulated active carbon obtained from carbonaceous powder, a spherical activated carbon prepared by burning an organic polymer, or a spherical activated carbon obtained from a petroleum hydrocarbon (a petroleum pitch).

The granulated active carbon obtained from carbonaceous powder can be prepared, for example, by the following procedure. A binder such as tar or pitch is used to granulate a carbonaceous powdery material. The resulting microspherical shaped substance is carbonized (or calcinated) by heat-treatment at 600 to 1000°C in an inert atmosphere. Further, the carbonized substance is activated to obtain the granulated active carbon. The activation may be performed by, for example, a steam-activation method, a chemical activation method, an air-activation method, or a $CO_2$ activation method. The steam-activation can be performed at 800 to 1100°C in an atmosphere of steam.

The spherical activated carbon prepared by burning an organic polymer is disclosed in, for example, Japanese Examined Patent Publication (Kokoku) No. 61-1366, and may be prepared by the following procedure. A condensation-type or polyaddition-type thermosetting prepolymer is mixed with a curing agent, a curing catalyst, and an emulsifying agent. The mixture is emulsified in water with stirring, and reacted at room temperature or at a higher temperature with stirring. The reaction system becomes a suspension, and then a spherical thermosetting polymer is generated with stirring. The product is collected, and heated at 500°C or more in an inert atmosphere to be carbonized. The carbonized substance is activated by the above-mentioned method to obtain the spherical activated carbon.

The spherical activated carbon obtained from a petroleum pitch has a diameter of preferably 0.05 to 2 mm, more preferably 0.1 to 1 mm, a specific surface area of preferably 500 to 2000 $m^2/g$, more preferably 700 to 1500 $m^2/g$, and a volume of pores having a pore radius of 100 to 75000 angstrom of preferably 0.01 to 1 mL/g. The spherical activated carbon obtained from a petroleum pitch may be prepared, for example, by the following two methods.

The first method is disclosed in, for example, Japanese Examined Patent Publication (Kokoku) No. 51-76 (United States Patent No. 3917806) or Japanese Unexamined Patent Publication (Kokai) No. 54-89010 (United States Patent No. 4761284). In the first method, a pitch is granulated under a melted condition, and treated with oxygen. The resulting infusible substance is heated at 600 to 1000°C in an inert atmosphere to be carbonized. Further, the carbonized substance is activated at 850 to 1000°C in an atmosphere of steam. The second method is disclosed in, for example, Japanese Examined Patent Publication (Kokoku) No. 59-10930 (United States Patent No. 4420433). In the second method, a pitch is formed into string-like shaped products under a melted condition. The string-like shaped products are broken and added to hot water to be spheroidized. An oxygen treatment is performed to obtain an infusible substance, and the carbonization and activation are carried out by the same methods as described in the first method.

As the spherical activated carbon which may be used as the active ingredient in the present invention, (1) a spherical activated carbon treated with ammonia, or (2) a spherical activated carbon treated with an oxidation treatment and/or a reduction treatment, may be used. The treatments can be applied to, for example, the spherical activated carbon obtained from a petroleum pitch, the granulated active carbon obtained from carbonaceous powder, or the spherical activated carbon prepared by burning an organic polymer, as described above.

In the ammonia treatment, for example, a spherical activated carbon is treated in an aqueous ammonia solution (1 to 1000 ppm; a volume ratio of the aqueous ammonia solution to the spherical activated carbon = 2 to 10) at 10 to 50°C for 0.5 to 5 hours. A spherical activated carbon obtained by treating a spherical activated carbon derived from a petroleum pitch with ammonia is disclosed in, for example, Japanese Unexamined Patent Publication (Kokai) No. 56-5313 (United States Patent No. 4761284). As the spherical activated carbon treated with ammonia, there may be mentioned, for example, a spherical activated carbon having a diameter of 0.05 to 2 mm, preferably 0.1 to 1 mm, a specific surface area of 500 to 2000 $m^2/g$, preferably 700 to 1500 $m^2/g$, a volume of pores having a pore radius of 100 to 75000 angstrom of 0.01 to 1 mL/g, and a pH of 6 to 8.

The above-mentioned oxidation treatment means a heat treatment at a high temperature in an oxidative atmosphere containing oxygen. As an oxygen source, for example, pure oxygen, nitrogen oxide, or air can be used. The above-mentioned reduction treatment means a heat treatment at a high temperature in an inert atmosphere with respect to carbon. The inert atmosphere with respect to carbon can be formed by using nitrogen gas, argon gas, or helium gas, or a mixed gas thereof.

The oxidation treatment is carried out in an atmosphere containing preferably 0.5 to 25% by volume, more preferably 3 to 10% by volume of oxygen at preferably 300 to 700°C, more preferably 400 to 600°C. The reduction treatment is carried out in an inert atmosphere at preferably 700 to 1100°C, more preferably 800 to 1000°C.

A spherical activated carbon obtained by treating a spherical activated carbon derived from a petroleum pitch with the oxidative treatment and/or the reduction treatment is disclosed in, for example, Japanese Examined Patent Publication (Kokoku) No. 62-11611 (United States Patent No. 4681764).

As the spherical activated carbon treated with the oxidative treatment and/or the reduction treatment, a spherical activated carbon having a diameter of 0.05 to 2 mm, preferably 0.1 to 1 mm, a specific surface area of 500 to 2000 $m^2/g$, preferably 700 to 1500 $m^2/g$, and a volume of pores having a pore radius of 100 to 75000 angstrom of 0.01 to 1 mL/g is preferable.

The spherical activated carbon disclosed in JP2002-308785 is a spherical activated carbon having a diameter of 0.01 to 1 mm, a specific surface area determined by a BET method of 700 $m^2/g$ or more, a volume of pores having a pore diameter of 20 to 15000 nm ranges from not less than 0.04 mL/g to less than 0.10 mL/g, a total amount of acidic groups of 0.30 to 1.20 meq/g, and a total amount of basic groups of 0.20 to 0.70 meq/g. The spherical activated carbon disclosed in JP2002-308785 has a specific range of pore volume, namely, a volume of pores having a pore diameter of 20 to 15000 nm is from not less than 0.04 mL/g to less than 0.10 mL/g. Further, a spherical activated carbon having a total amount of basic groups of 0.20 to 1.00 meq/g (see Japanese Patent Application No. 2002-293906 or Japanese Patent Application No. 2002-293907) may be used in the present invention.

In the spherical activated carbon disclosed in JP11-292770, a volume of pores having a pore radius of 100 to 75000 angstrom, i.e., a volume of pores having a pore diameter of 20 to 15000 nm, is 0.1 to 1 mL/g. According to the disclosures in JP2002-308785, when the volume of pores having a pore diameter of 20 to 15000 nm is adjusted to a range of from not less than 0.04 mL/g to less than 0.10 mL/g, an adsorbability of α-amylase that is a useful substance, is significantly lowered, while maintaining a high adsorbability of β-aminoisobutyric acid, that is a toxic substance. When the volume of pores having a pore diameter of 20 to 15000 nm is increased, the useful substances such as digestive enzymes are more easily adsorbed. Therefore, a smaller volume of pores having a pore diameter of 20 to 15000 nm is preferable from a viewpoint that an adsorption of useful substances is reduced. On the other hand, if the volume of pores having such a pore diameter becomes too small, the adsorption of harmful substances is lowered. Therefore, in the adsorbent for an oral administration, a ratio (T/U) of an adsorption amount (T) of toxic substances to an adsorption amount (U) of useful substances, that is, a selective adsorption rate, is important. For example, the selective adsorption rate of the spherical activated carbon can be evaluated by the ratio (Tb/Ua) of an adsorption amount (Tb) of DL-β-aminoisobutyric acid (toxic substance) to an adsorption amount (Ua) of α-amylase (useful substance). More particularly, the selective adsorption rate can be evaluated by, for example, an equation:

$$A\ =\ Tb/Ua$$

wherein A denotes a selective adsorption rate, Tb denotes an adsorption amount of DL-β-aminoisobutyric acid, and Ua denotes an adsorption amount of a-amylase.

The spherical activated carbon disclosed in JP2002-308785 exhibits an excellent selective adsorption rate when the volume of pores having a pore diameter of 20 to 15000 nm ranges from not less than 0.04 mL/g to less than 0.10 mL/g, and a more excellent selective adsorption rate when the volume of pores having a pore diameter of 20 to 15000 nm ranges from not less than 0.05 mL/g to less than 0.10 mL/g.

The spherical activated carbon disclosed in JP2002-308785 has a diameter of 0.01 to 1 mm, preferably 0.02 to 0.8 mm. In this connection, the expression that "a diameter is Dl to Du" as used herein means that a screen passing percentage (%) in a range of a screen opening Dl to Du is 90% or more in a particle-sizes accumulating standard curve prepared in accordance with JIS K 1474 as mentioned below in relation to a method for determining an average particle diameter.

The spherical activated carbon disclosed in JP2002-308785 has a specific surface area (referred to as "SSA" hereinafter) determined by a BET method of 700 $m^2/g$ or more. When the spherical activated carbon has an SSA of less than 700 $m^2/g$, an adsorbability of toxic substances is lowered. The SSA is preferably 800 $m^2/g$ or more. The upper limit of the SSA is not particularly limited, but the SSA is preferably 2500 $m^2/g$ or less in view of a bulk density and strength.

The spherical activated carbon disclosed in JP2002-308785 has a special constitution of functional groups, that is, a total amount of acidic groups is 0.30 to 1.20 meq/g, and a total amount of basic groups is 0.20 to 0.70 meq/g. When the spherical activated carbon does not satisfy the functional-groups requirement, i.e., the total amount of acidic groups is 0.30 to 1.20 meq/g and the total amount of basic groups is 0.20 to 0.70 meq/g, the adsorbability of the harmful substances is lowered. In the functional-groups requirement, the total amount of acidic groups is preferably 0.30 to 1.00 meq/g and the total amount of basic groups is preferably 0.30 to 0.60 meq/g. A preferable functional-groups constitution is that the total amount of acidic groups is 0.30 to 1.20 meq/g, the total amount of basic groups is 0.20 to 0.70 meq/g, a phenolic hydroxyl group is 0.20 to 0.70 meq/g, and a carboxyl group is 0.15 meq/g or less, and a ratio (a/b) of the total amount of acidic groups (a) to the total amount of basic groups (b) is 0.40 to 2.5, and a relation [(b+c)-d] between the total amount of basic groups (b), the phenolic hydroxyl group (c), and the carboxyl group (d) is 0.60 or more.

The spherical activated carbon disclosed in JP2002-308785 may be prepared by, for example, the following methods.

First, a dicyclic or tricyclic aromatic compound or a mixture thereof having a boiling point of 200°C or more is added as an additive to a pitch such as a petroleum pitch or a coal pitch. The whole is heated and mixed, and then shaped to obtain a shaped pitch. The spherical activated carbon is for oral administration, and the raw material must have a sufficient purity from a safety standpoint, and have stable properties.

Thereafter, the shaped pitch is dispersed and granulated in hot water at 70 to 180°C, with stirring, to obtain a microspherical shaped pitch. Further, the additive is extracted and removed from the shaped pitch by a solvent having a low solubility to the pitch but a high solubility to the additive. The resulting porous pitch is oxidized by an oxidizing agent to obtain a porous pitch having an infusibility to a heat. The resulting infusible porous pitch is treated at 800 to 1000°C in a gas flow such as steam or carbon dioxide gas reactive with carbon to obtain a porous carbonaceous substance.

Then, the resulting porous carbonaceous substance is oxidized in a temperature range of from 300 to 800°C, preferably 320 to 600°C in an atmosphere containing 0.1 to 50% by volume, preferably 1 to 30% by volume, particularly preferably 3 to 20% by volume of oxygen, and thereafter reduced in a temperature range of from 800 to 1200°C, preferably 800 to 1000°C, in an atmosphere of a non-oxidizable gas to obtain the spherical activated carbon disclosed in JP2002-308785.

In the above method, the atmosphere containing oxygen in the particular amount may be pure oxygen, or nitrogen oxides or air as the oxygen source. As the atmosphere inert against carbon, for example, nitrogen, argon or helium may be used alone or in the form of a mixture thereof.

The purpose of the addition of the aromatic compound to the raw pitch is that a flowability of the raw pitch is enhanced by lowering a softening point of the raw pitch whereby the granulation thereof is made easier, and the porous pitch is produced by extracting and removing the additive from the shaped pitch, whereby a structure control and a calcination of the carbonaceous material by oxidization in the subsequent steps is made easier. As the additive, for example, naphthalene, methylnaphthalene, phenyl-naphthalene, benzyl-naphthalene, methylanthracene, phenanthrene, or biphenyl may be used alone or in a mixture thereof. An amount of the additive added to the pitch is preferably 10 to 50 parts by weight of the aromatic compound with respect to 100 parts by weight of the pitch.

It is preferable that the pitch and the additive are mixed under a melted condition with heating, to achieve a homogeneous mixing. Further, it is preferable that the mixture of the pitch and the additive is shaped to form particles having a particle size of about 0.01 to 1 mm, to control the particle size (diameter) of the resulting porous spherical carbonaceous substance. The shaping may be conducted during the melted condition, or by grinding the mixture after it has been cooled.

A preferable solvent used to extract and remove the additive from the mixture of the pitch and the additive may be, for example, an aliphatic hydrocarbon, such as butane, pentane, hexane, or heptane, a mixture comprising an aliphatic hydrocarbon as a main component, such as naphtha or kerosene, or an aliphatic alcohol, such as methanol, ethanol, propanol, or butanol.

The additive may be removed from the shaped mixture by extracting the additive with the solvent from the shaped mixture of the pitch and the additive, while maintaining the shape. It is assumed that, upon the extraction, through-holes of the additive are formed in the shaped product, and a shaped pitch having a uniform porosity can be obtained. In this connection, the size of through-holes of the additive (i.e., pore volume) may be controlled by a conventional method, for example, by controlling an amount of the additive, or a precipitating temperature (cooling temperature) of the additive in the granulating step of the shaped pitch. Further, when the resulting shaped pitch is crosslinked by oxidation, the pore volume generated by extracting the additive is affected by a condition of the treatment. For example, if it is strongly crosslinked by oxidation, a heat contraction caused by a heat treatment is small, and thus the pores obtained by extracting the additive tend to be maintained.

Then, the resulting porous shaped pitch is crosslinked by oxidation, that is, the resulting porous shaped pitch is oxidized by an oxidizing agent, preferably at room temperature to 300°C to obtain the porous infusible shaped pitch having a non-fusibility to heat. As the oxidizing agent, for example, oxygen gas $(O_2)$, or a gas mixture prepared by diluting oxygen gas $(O_2)$ with air or nitrogen may be used.

Properties of the spherical activated carbon disclosed in JP2002-308785, namely, the average particle diameter, the specific surface area, the pore volume, the total amount of acidic groups, and the total amount of basic groups are measured by the following methods.

(1) Average particle diameter

A particle-sizes accumulating standard curve is prepared in accordance with JIS K 1474 for the spherical activated carbon. The average particle diameter is determined from a screen opening (mm) at an intersection point with a line that is horizontal to an abscissa axis and starts from an intersection point in the particle-sizes accumulating standard curve with a perpendicular line from a 50% point of the abscissa axis.

(2) Specific surface area

An amount of gas adsorbed is measured by a specific surface area measuring apparatus (for example, Flow Sorb II 2300 manufactured by MICROMERITICS) in accordance with a gas adsorbing method of a continuous flow for the spherical activated carbon sample, and a specific surface area can be calculated by a BET equation. More particularly, the spherical activated carbon is charged as a sample in a sample tube. A helium gas stream containing 30% by volume of nitrogen is passed through the sample tube, and an amount of nitrogen adsorbed to the spherical activated carbon sample is measured by the following procedures. Specifically, the sample tube is cooled to -196°C, whereby nitrogen is adsorbed to the spherical activated carbon sample, and then the temperature of the sample tube is raised to room temperature. During the raising of the temperature, nitrogen is emitted from the spherical activated carbon sample. The amount of nitrogen emitted is measured by a heat conductivity type detector as an amount (v) of gas adsorbed.

A value $v_m$ is calculated in accordance with a one-point method (relative pressure x = 0.3) by a nitrogen adsorption at a temperature of liquid nitrogen, using an approximate equation:

$$v_m = 1/(v \cdot (1-x))$$

derived from the BET equation. Then, a specific surface area of the sample is calculated by an equation:

$$\text{specific surface area} = 4.35 \times v_m (m^2/g).$$

In the above equations, $v_m$ is an adsorption amount ($cm^3/g$) necessary to form a monomolecular layer on a surface of the sample, v is an adsorption amount ($cm^3/g$) actually found, and x is a relative pressure.

(3) Pore volume by a mercury injection method

The pore volume can be measured by a mercury press-injection porosimeter (for example, AUTOPORE 9200 manufactured by MICROMERITICS). The spherical activated carbon is charged as a sample in a sample vessel, and degassed under a pressure of 2.67Pa or less for 30 minutes. Then, mercury is introduced into the sample vessel, and a pressure applied is gradually increased (maximum pressure = 414 MPa) to force the mercury into the micropores in the spherical activated carbon sample. A pore volume distribution of the spherical activated carbon sample is measured from a relationship between the pressure and an amount of forced mercury by equations as given below. Specifically, a volume of mercury inserted into the spherical activated carbon sample while a pressure is applied is increased from a pressure (0.07 MPa) corresponding to a pore diameter of 15 um to the maximum pressure (414 Mpa) corresponding to a pore diameter of 3 nm. A pore diameter can be calculated as follows. When mercury is forced into a cylindrical micropore having a diameter (D) by applying a pressure (P), a surface tension (γ) of mercury is balanced with a pressure acting on a section of the micropore, and thus, the following equation is held:

$$-\pi D \gamma \cos\theta = \pi (D/2)^2 \cdot P$$

wherein θ is a contact angle of mercury and a wall of the micropore. Therefore, the following equation:

$$D = (-4\gamma\cos\theta)/P$$

is held.

In the present specification, the relationship between the pressure (P) and the pore diameter (D) is calculated by an equation:

$$D = 1.27/P$$

given that a surface tension of mercury is 484 dyne/cm, a contact angle of mercury and carbon is 130°, a unit of the pressure P is Mpa, and a unit of the pore diameter D is $\mu$m. The volume of pores having a pore diameter of 20 to 15000 nm in the present invention corresponds to a volume of mercury inserted by applying a pressure increasing from 0.07 Mpa to 63.5 Mpa.

(4) Total amount of acidic groups
The total amount of acidic groups is an amount of NaOH consumed, which may be determined by adding 1 g of the spherical activated carbon sample, after being crushed to form particles having a size of less than 200 mesh, to 50 mL of a 0.05N NaOH solution; shaking the mixture for 48 hours; then filtering out the spherical activated carbon sample; and titrating until neutralization.

(5) Total amount of basic groups
The total amount of basic groups is an amount of HCl consumed, which may be determined by adding 1 g of the spherical activated carbon sample after being crushed to form particles having a less than 200 mesh size, to 50 mL of a 0.05N HCl solution; shaking the mixture for 24 hours; then filtering out the spherical activated carbon sample; and titrating until neutralization.

As the spherical activated carbon, which is an effective ingredient of the medicament according to the present invention, a spherical activated carbon having a small average particle diameter disclosed in Japanese Patent Application No. 2004-110575, that is, a spherical activated carbon having an average diameter of 50 to 200 $\mu$m, and a specific surface area of 700 m$^2$/g or more determined by a BET method, or a surface-modified spherical activated carbon having a small average particle diameter disclosed in Japanese Patent Application No. 2004-110576, that is, a surface-modified spherical activated carbon wherein an average diameter is 50 to 200 $\mu$m, a specific surface area determined by a BET method is 700 m$^2$/g or more, a total amount of acidic groups is 0.30 to 1.20 meq/g, and a total amount of basic groups is 0.20 to 0.9 meq/g, can be used.

The medicament according to the present invention is effective for treating or preventing a diabetic neuropathy which is a diabetic complication. For example, a repetitive oral administration does not cause toxicity, or harmful side effects such as constipation.

As concretely shown in the Examples mentioned below, when the spherical activated carbon is orally administered to GK rats, which are known as a spontaneous type 2 diabetic model rat, a motor nerve conduction velocity (MNCV) can be significantly improved, while a blood glucose level is not lowered. This apparently means that the spherical activated carbon is effective for treating or preventing a diabetic neuropathy.

The spherical activated carbon (preferably having a particle size of 0.01 to 2 mm) which may be used as the active ingredient of the medicament according to the present invention may be administered alone or, optionally, together with a pharmaceutically or veterinary acceptable ordinary carrier or diluent, to a subject (an animal, preferably a mammal, particularly a human) in need of a treatment or prevention of a diabetic neuropathy, in an amount effective thereof. Preferably, the medicament according to the present invention may be orally administered. The dose depends on, for example, the kind of subject (a mammal, particularly a human), the age, individual differences, and/or symptoms of the subject. For example, when the subject is a human, the dose is normally 0.2 to 20 g of spherical activated carbon per day. The dose may be appropriately changed in accordance with the symptoms. Further, the dose may be administered as a single dose or a multiple dose. The spherical activated carbon per se may be administered, or it may be administered as a pharmaceutical composition containing the spherical activated carbon. In the former, the spherical activated carbon may be administered as a slurry prepared by suspending it in drinking water.

The formulation may be administered in any form, such as granules, tablets, sugar-coated tablets, capsules, sticks, divided packages, or suspensions. In the case of capsules, the usual gelatin capsules, or if necessary, enteric capsules may be used. In the case of granules, tablets, or sugar-coated tablets, the formulations must be broken into the original fine particles inside the body.

The content of the spherical activated carbon in the medicament is normally 1 to 100%. The preferred medicaments are capsules, sticks, or divided packages, and the spherical activated carbon per se may be packed into a package.

EXAMPLES

The present invention now will be further illustrated by, but is by no means limited to, the following Examples.

Preparation Example 1: Preparation of porous spherical carbonaceous substance

A method as described in Example 1 of Japanese Patent No. 3522708 (Japanese Unexamined Patent Publication (Kokai) No. 2002-308785) was used to obtain a porous spherical carbonaceous substance. Concrete procedures were as follows.
Petroleum pitch (68 kg) (softening point = 210°C; quinoline insoluble contents = 1% or less by weight; ratio of hydrogen atoms/carbon atoms = 0.63) and naphthalene (32 kg) were charged into an autoclave (internal volume = 300 L) equipped with stirring fans, melted at 180°C, and mixed. The mixture was extruded at 80 to 90°C to form string-like shaped products. Then, the string-like shaped products were broken so that a ratio of a diameter to a length became about 1 to 2.
The resulting broken products were added to an aqueous solution containing 0.23% by weight of polyvinyl alcohol (saponification value = 88%) and heated to 93°C, and dispersed with stirring to be spheroidized. Then, the whole was cooled by replacing the polyvinyl alcohol aqueous solution with water, at 20°C for 3 hours, whereby the pitch was solidified and naphthalene crystals were precipitated, and a slurry of spherical shaped products of pitch was obtained.
After most of the water was removed by filtration, naphthalene in pitch was extracted and removed with n-hexane at an amount about 6 times that of the spherical shaped products of pitch. The resulting porous spherical pitch was heated to 235°C by passing a heated air in a fluidized bed, and allowing to stand at 235°C for 1 hour to be oxidized, and a porous spherical oxidized pitch was obtained, which is non-fusible to heat.
Thereafter, the resulting porous spherical oxidized pitch was activated in a fluidized bed at 900°C for 170 minutes by a nitrogen gas atmosphere containing 50% by volume of steam to obtain a porous spherical activated carbon. Further, the resulting spherical activated carbon was oxidized in a fluidized bed at 470°C for 3 hours and 15 minutes by a nitrogen-oxygen atmosphere containing 18.5% by volume of oxygen, and reduced in a fluidized bed at 900°C for 17 minutes by a nitrogen gas atmosphere, to obtain a porous spherical carbonaceous substance. The resulting porous spherical carbonaceous substance was used as a spherical activated carbon in Pharmacological Experiments as mentioned below. The main properties of the resulting carbonaceous substance are as follows:

Specific surface area: 1300 $m^2$/g (a BET method);
Pore volume: 0.08 mL/g
(The pore volume was determined by a mercury injection method and corresponds to a volume of pores having a diameter of 20 to 15000 nm);
Average particle diameter: 350 $\mu$m;
Total amount of acidic groups: 0.67 meq/g; and
Total amount of basic groups: 0.54 meq/g.

Examples of Pharmacological Experiments

(a) Methods of Experiments
GK/Jcl (Goto-Kakizaki/Jcl) rats, which are spontaneous type 2 diabetic model rats usually used for a diabetic neuropathy, were used as test animals. Ten GK/Jcl rats (male, 20 weeks old) were purchased from Clea Japan Inc. After the rats were fed and tamed, the rats were divided into two groups; a control group (DC) consisting of 5 rats and a spherical activated carbon-administering group (DX) consisting of 5 rats such that the rats are not biased between two groups with respect to a body weight, a blood glucose level, HbA1c, and a motor nerve conduction velocity (MNCV) as an index of a diabetic neuropathy. Immediately after the division into the groups, a methylcellulose aqueous solution (MC) was forcedly administered per os to the control group five times per week. To the spherical activated carbon-administering group (DX), on the other hand, a mixture of an MC solution and 26.7% (W/V) of the spherical activated carbon prepared in Preparation Example 1 was forcedly administered per os at an amount of 4 g/kg weight five times per week. Each group was observed for 8 weeks.
A latex aggregating method (DCA2000 system: Sankyo Co., Ltd.) was used to measure HbA1c, and Neuropack $\mu$ (Nihon Kohden Corporation) was used to measure a motor nerve conduction velocity (MNCV).

(b) Results of the Experiments

After 4 weeks or 8 weeks from the beginning of the experiment, body weights, blood glucose levels, HbA1cs, and motor nerve conduction velocities (MNCVs) were measured to evaluate a diabetic neuropathy. Table 1 shows the results when the groups were divided, Table 2 shows the results at 4 weeks later, Table 3 shows the results at 8 weeks later, and Table 4 shows rates (ΔMNCV) of changes in a motor nerve conduction velocity (MNCV). In Tables 1 to 4, "NS" means that no significant difference was observed at a T-test.

Table 1

| When the groups were divided | | | | |
|---|---|---|---|---|
| Groups | Body weight (g) | Blood glucose level (mg/dL) | HbA1c (%) | MNCV (m/sec) |
| Diabetes + methylcellulose administration(control group) | 323±21 | 245±35 | 4.3±0.6 | 59±1 |
| Diabetes + spherical activated carbon administration (spherical activated carbon-administrating group) | 323±13 | 245±24 | 4.2±0.3 | 58±2 |
| T-test | NS | NS | NS | NS |
| (Mean±SD) | | | | |

Table 2

| 4 weeks from start of administration | | | | |
|---|---|---|---|---|
| Groups | Body weight (g) | Glu (mg/dL) | HbA1c (%) | MNCV (m/sec) |
| Diabetes + methylcellulose administration (control group) | 343±20 | 250±34 | 4.2±0.4 | 58±3 |
| Diabetes + spherical activated carbon administration (spherical activated carbon-administrating group) | 347±15 | 253±53 | 4.0±0.4 | 61±1 |
| T-test | NS | NS | NS | p<0.05 |
| (Mean±SD) | | | | |

Table 3

| 8 weeks from start of administration | | | | |
|---|---|---|---|---|
| Groups | Body weight (g) | Glu (mg/dL) | HbA1c (%) | MNCV (m/sec) |
| Diabetes + methylcellulose administration (controlgroup) | 354±18 | 279±31 | 4.0±0.3 | 57±2 |
| Diabetes + spherical activated carbon administration (spherical activated carbon-administrating group) | 362±16 | 244±22 | 3.9±0.3 | 62±1 |

(continued)

| 8 weeks from start of administration | | | | |
|---|---|---|---|---|
| Groups | Body weight (g) | Glu (mg/dL) | HbA1c (%) | MNCV (m/sec) |
| T-test | NS | NS | NS | p<0.01 |
| (Mean±SD) | | | | |

Table 4

| Rate of change in motor nerve conduct i on velocity (ΔMNCV) | | | |
|---|---|---|---|
| Groups | 0 week/0 week | 4 weeks/0 week | 8 weeks/4 weeks |
| Diabetes + methylcellulose administration(control group) | 1.00±0.00 | 0.97±0.06 | 0.99±0.03 |
| Diabetes + spherical activated carbon administration (spherical activated carbon-administrating group) | 1.00±0.00 | 1.05±0.04 | 1.01±0.03 |
| T-test | - | p<0. 05 | NS |
| (Mean±SD) | | | |

As shown in Tables 1 to 4, in comparison with the control group, the spherical activated carbon-administering group showed a significant increase or improvement in a delay (drop) of a motor nerve conduction velocity, whereas the body weight, blood glucose level, and an HbA1c were not changed during the experimental period. Therefore, an advantageous effect on a diabetic neuropathy or a diabetic disorder of a peripheral nerve system was confirmed.

Formulation Example 1: Preparation of Capsules

Capsules were prepared by encapsulating 200 mg of the spherical activated carbon prepared in Preparation Example 1 into gelatin capsules.

Formulation Example 2: Preparation of Stick-type Sachet

A stick-type sachet was prepared by filling 2 g of the spherical activated carbon prepared in Preparation Example 1 into laminated film sticks and heat-sealing the sticks.

INDUSTRIAL APPLICABILITY

The anti-diabetic neuropathy agent according to the present invention is effective in treating or preventing a diabetic neuropathy, which is a diabetic complication.
Although the present invention has been described with reference to specific embodiments, various changes and modifications obvious to those skilled in the art are possible without departing from the scope of the appended claims.

**Claims**

1. An agent for treating or preventing a diabetic neuropathy, comprising a spherical activated carbon as an effective component.

2. The agent for treating or preventing a diabetic neuropathy according to claim 1, wherein it is an agent for an oral administration.

3. The agent for treating or preventing a diabetic neuropathy according to claim 1 or 2, wherein a particle size of the spherical activated carbon is 0.01 to 2 mm.

4. The agent for treating or preventing a diabetic neuropathy according to any one of claims 1 to 3, wherein it is an agent for ameliorating a motor nerve conduction velocity.

5. A pharmaceutical composition for treating or preventing a diabetic neuropathy, comprising a spherical activated carbon and a pharmaceutically or veterinary acceptable carrier or diluent.

6. The pharmaceutical composition for treating or preventing a diabetic neuropathy according to claim 5, wherein it is an agent for an oral administration.

7. The pharmaceutical composition for treating or preventing a diabetic neuropathy according to claim 5 or 6, wherein a particle size of the spherical activated carbon is 0.01 to 2 mm.

8. The pharmaceutical composition for treating or preventing a diabetic neuropathy according to any one of claims 5 to 7, wherein it is an agent for ameliorating a motor nerve conduction velocity.

9. A method for treating or preventing a diabetic neuropathy, comprising administrating to a subject in need thereof a spherical activated carbon in an amount effective thereof.

10. The method for treating or preventing a diabetic neuropathy according to claim 9, wherein the spherical activated carbon is orally administered.

11. The method for treating or preventing a diabetic neuropathy according to claim 9 or 10, wherein a particle size of the spherical activated carbon is 0.01 to 2 mm.

12. The method for treating or preventing a diabetic neuropathy according to any one of claims 9 to 11, wherein it is an agent for ameliorating a motor nerve conduction velocity.

13. Use of a spherical activated carbon for preparing a pharmaceutical composition for treating or preventing a diabetic neuropathy.

14. The use according to claim 13, wherein the pharmaceutical composition is for oral administration.

15. The use according to claim 13 or 14, wherein a particle size of the spherical activated carbon is 0.01 to 2 mm.

16. The use according to any one of claims 13 to 15, wherein the pharmaceutical composition is for ameliorating a motor nerve conduction velocity.

| **INTERNATIONAL SEARCH REPORT** | International application No. |
|---|---|
| | PCT/JP2005/017356 |

| A. CLASSIFICATION OF SUBJECT MATTER |
|---|
| **_A61K33/44_**(2006.01), **_A61P3/10_**(2006.01), **_A61P25/00_**(2006.01) |

According to International Patent Classification (IPC) or to both national classification and IPC

| B. FIELDS SEARCHED |
|---|
| Minimum documentation searched (classification system followed by classification symbols)<br>A61K33/44, A61P3/10, 25/00 |

Documentation searched other than minimum documentation to the extent that such documents are included in the fields searched

| Jitsuyo Shinan Koho | 1922-1996 | Jitsuyo Shinan Toroku Koho | 1996-2005 |
|---|---|---|---|
| Kokai Jitsuyo Shinan Koho | 1971-2005 | Toroku Jitsuyo Shinan Koho | 1994-2005 |

Electronic data base consulted during the international search (name of data base and, where practicable, search terms used)
MEDLINE/CAPLUS/EMBASE/BIOSIS(STN), JMEDPLUS(JOIS)

| C. DOCUMENTS CONSIDERED TO BE RELEVANT | | |
|---|---|---|
| Category* | Citation of document, with indication, where appropriate, of the relevant passages | Relevant to claim No. |
| X | JP 06-298653 A (Kureha Chemical Industry Co., Ltd.),<br>25 October, 1994 (25.10.94),<br>Full text<br>& EP 620006 A2  & US 5380526 A | 1-8,13-16 |
| X | JP 11-292770 A (Kureha Chemical Industry Co., Ltd.),<br>26 October, 1999 (26.10.99),<br>Full text<br>(Family: none) | 1-8,13-16 |

| ☒ Further documents are listed in the continuation of Box C. | ☐ See patent family annex. |
|---|---|

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>20 December, 2005 (20.12.05) | Date of mailing of the international search report<br>27 December, 2005 (27.12.05) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2005/017356

| C (Continuation). DOCUMENTS CONSIDERED TO BE RELEVANT | | |
|---|---|---|
| Category* | Citation of document, with indication, where appropriate, of the relevant passages | Relevant to claim No. |
| A | WO 2004/039380 A1 (Kureha Chemical Industry Co., Ltd.), 13 May, 2004 (13.05.04), Full text & EP 1547605 A1 & JP 3672200 B2 & US 2005/152890 A1 | 1-8,13-16 |

Form PCT/ISA/210 (continuation of second sheet) (April 2005)

**INTERNATIONAL SEARCH REPORT**

| International application No. |
| --- |
| PCT/JP2005/017356 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 9-12
   because they relate to subject matter not required to be searched by this Authority, namely:
   Claims 9 to 12 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7223955 A
- JP 11106352 A
- WO 01068136 A
- JP 6298653 A
- JP 11292770 A
- JP 3522708 B
- JP 2002308785 A
- JP 61001366 A
- JP 51000076 A
- US 3917806 A
- JP 54089010 A
- US 4761284 A
- JP 59010930 A
- US 4420433 A
- JP 56005313 A
- JP 62011611 A
- US 4681764 A
- JP 2002293906 A
- JP 2002293907 A
- JP 2004110575 A
- JP 2004110576 A

**Non-patent literature cited in the description**

- Igaku-no-Ayumi (Journal of Clinical and Experimental Medicine), Tonyobyo-Taishashokogun (Diabetes and Metabolic syndrome), State of Arts, 2004-2006. **JIRO NAKAMURA.** Treatment of diabetic neuropathy. Ishiyaku Publishers, Inc, 29 November 2003, 467-470